# DEMANDE DE BREVET EUROPEEN

(11) **EP 1 421 943 A1**
(43) Date de publication de la demande: **26.05.2004**
(21) Numéro de dépôt: 04003133.8
(22) Date de dépôt: 25.05.1998
(51) Int. Cl.: A61K 31/715

(54) **Utilisation des heparines de bas poids moléculaire pour la prévention et le traitement du trauma du système nerveux central**

(30) Priorité: 28.05.1997 FR 9706550
(62) Demande divisionnaire de: 98925773.8
(71) Demandeur: Aventis Pharma S.A., 92160 Antony (FR)
(72) Inventeur: Stutzmann, Jean-Marie, 94440 Villecresnes (FR); Uzan, André, 75116 Paris (FR); Wahl, Florence, 92100 Boulogne (FR)
(74) Mandataire: Rousseau, Pierrick Edouard

(57) **Abrégé**

Utilisation des héparines de bas poids moléculaire pour la prévention et le traitement du trauma du système nerveux central et notamment du traumatisme spinal, crânien ou crânio-spinal.

## Description

La présente invention concerne l'utilisation des héparines de bas poids moléculaire pour la prévention et le traitement du trauma du système nerveux central et notamment du traumatisme spinal, crânien ou crânio-spinal.

L'invention concerne également l'utilisation des héparines de bas poids moléculaire pour la préparation d'un médicament utile pour la prévention et le traitement du trauma du système nerveux central et notamment du traumatisme spinal, crânien ou crânio-spinal.

L'héparine standard est un polysaccharide sulfaté de poids moléculaire moyen de 12000-15000 daltons isolé des muqueuses intestinales de boeuf, de mouton et de porc. L'héparine est utilisée cliniquement pour la prévention et le traitement des désordres thromboemboliques mais cause parfois des hémorragies.

Depuis une dizaine d'années, l'héparine est progressivement remplacée par les héparines de bas poids moléculaire qui ne présentent plus ou à un degré moindre l'inconvénient de faire saigner et ne nécessitent plus qu'une injection par jour au lieu de 2 à 3 injections par jour pour l'héparine standard. Ces héparines de bas poids moléculaire sont préparées notamment par fractionnement, dépolymérisation controlée de l'héparine ou par synthèse chimique. Elles présentent un rapport activité anti Xa/activité anti lla supérieur à 2.

Il a maintenant été trouvé que les héparines de bas poids moléculaire réduisent la taille du trauma du système nerveux central et notamment du traumatisme spinal, crânien ou crânio-spinal.

Les traumatismes du système nerveux central (trauma SNC ou neurotrauma) concernent le trauma du cerveau (trauma cérébral) et les trauma de la moelle épinière (trauma médullaire). Ils sont la conséquence d'un choc au niveau du système nerveux central (accident de voiture, moto, ski, piscine..) souvent mais pas toujours accompagnés de fractures. Les conséquences de ces trauma sont des désordres neurologiques tels que épilepsie, altération de la conscience, problèmes moteur, amnésie, aggressivité et déficit psychoaffectif.

Selon l'invention, on utilise de préférence une héparine de bas poids moléculaire ayant un poids moléculaire moyen compris entre 1000 et 10000 daltons, notamment entre 1500 et 6000 daltons et, en particulier, entre 4000 et 5000 daltons.

Elles peuvent être préparées par différents procédés à partir de l'héparine :
- fractionnement au moyen de solvants (FR2440376, US 4692435),
- fractionnement sur résine anionique (FR2453875),
- gel filtration (BARROWCLIFFE, Thromb. Res. 12, 27-36 (1977),
- Chromatographie d'affinité (US4401758),
- dépolymérisation controlée au moyen d'un agent chimique : acide nitreux (EP14184, EP37319, EP76279, EP623629, FR2503714, US4804652; WO813276), β élimination à partir d'un ester de l'héparine (EP40144, US5389618), périodate (EP287477), borohydrure de sodium (EP347588, EP380943), acide ascorbique (US 4533549); peroxyde d'hydrogène (US4629699, US4791195), hydroxyde d'ammonium quaternaire à partir d'un sel d'ammonium quaternaire d'héparine (US4981955), hydroxyde de métal alcalin (EP380943, EP347588) ou par voie enzymatique (EP64452, US4396762, EP244235, EP244236; US 4826827; US 3766167); au moyen d'irradiation (EP 269981).

Certaines peuvent également être préparées par synthèse chimique (US4801583, US4818816, EP165134, EP84999, FR2535306)

Parmi ces héparines de bas poids moléculaire, on peut citer plus particulièrement l'énoxaparine (DCI) commercialisée par RHONE-POULENC RORER, la nadroparine (DCI) commercialisée par SANOFI, la parnaparine (DCI) commercialisée par OPOCRIN-ALFA, la reviparine (DCl) commercialisée par KNOLL, la daltéparine (DCI) commercialisée par KABI PHARMACIA, la tinzaparine (DCI) commercialisée par NOVO NORDISK, la danaparoide (DCI) commercialisée par ORGANON, l'ardeparine (DCI) développée par WYETH AYERST, la certoparine (DCI) commercialisée par SANDOZ et les produits en étude tels que le CY222 de SANOFI-CHOAY (Thromb. Haemostasis, 58 (1), 553 (1987)), le SR90107/ORG31540 de SANOFI-ORGANON (Thrombosis and Haemostasis, 74, 1468-1473 (1995)).

Préférentiellement, les héparines de bas poids moléculaire sont constituées d'oligosaccharides ayant un acide 2-O-sulfo-4-énopyranosuronique à l'une de leurs extrémités.

Une héparine de bas poids moléculaire particulièrement avantageuse est obtenue par dépolymérisation d'un ester de l'héparine au moyen d'une base telle que la soude.

L'effet des héparines de bas poids moléculaire sur le trauma du système nerveux central est démontré chez le rat sur le trauma induit selon la technique suivante : des rats mâles Sprague-Dawley (Charles River France) pesant 280-300 g (13 pour le groupe témoin et 13 pour le groupe traité) sont anesthésiés à l'halothane (1,5%) dans un mélange N₂O/O₂ (70/30) et placés en contention stéréotaxique. L'épicrâne est incisé et un trou est effectué au moyen d'une perceuse dentée au niveau du cortex pariétal droit (coordonnées : 3,5 mm antérieur à 6 mm au-dessus de la ligne interaurale). Un tube de polyéthylène de 3 mm de diamètre intérieur est placé sur la dure mère, fixé dans la cavité crânienne avec du ciment dentaire et relié à une valve solénoïde (Danfoss Evsi 24V, 15W). La dure mère est gardée intacte. La valve est reliée à une pompe HPLC (Walters 590). Le système est rempli d'eau stérile et quand la pompe a atteint une pression de 3,8 à 4 bar, la percussion fluide de sévérité modérée (1,6-1,8 bar) est induite par une brève ouverture (20 ms) de la valve. Le tube est ensuite retiré, l'incision suturée et les animaux sont remis dans leur cage dans une pièce chauffée à 26-28°C.

Les héparines de bas poids moléculaire dissoutes dans une solution saline (NaCl 0,9%) sont administrées de la manière suivante :
2 heures après la lésion : 0,5mg/kg/5ml IV bolus,
2 heures 15 minutes après la lésion : 1mg/kg/5ml SC,
6 heures après la lésion : 1mg/kg/5ml SC,
24 heures après la lésion : 1mg/kg/5ml SC et
30 heures après la lésion : 1mg/kg/5ml SC.

Au groupe témoin on administre 5ml/kg d'une solution saline (NaCl 0,9%) dans les mêmes conditions.

Les animaux sont sacrifiés une semaine après le trauma et la taille du trauma est évaluée histologiquement. Des sections coronales sont colorées avec un mélange hématoxylin/éosine et les aires du trauma sont mesurées avec un analyseur d'image.

Dans ce test, les héparines de bas poids moléculaire réduisent d'au moins 40% la taille du trauma cérébral.

L'énoxaparine (LOVENOX^{R}) réduit de 50% la taille du trauma cérébral.

Les médicaments sont constitués par un sel (sodium ou calcium de préférence) d'une héparine de bas poids moléculaire sous forme d'une composition dans laquelle il est associé à tout autre produit pharmaceutiquement compatible, pouvant être inerte ou physiologiquement actif. Les médicaments selon l'invention peuvent être employés par voie intraveineuse, sous-cutanée, orale, rectale, topique ou pulmonaire (inhalation).

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme compositions solides pour administration orale, peuvent être utilisés des comprimés, des pilules, des poudres (capsules de gélatine, cachets) ou des granulés. Dans ces compositions, le principe actif est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un agent favorisant l'absorption orale, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,2 mg et 4 mg par kg par jour par voie sous-cutanée soit 14 à 280 mg par jour pour un adulte.

D'une façon générale, le médecin déterminera la posologie appropriée en fonction de l'âge, du poids et de tous les autres facteurs propres au sujet à traiter.

La présente invention concerne également la méthode de prévention ou de traitement du trauma du système nerveux central et notamment du traumatisme spinal, crânien ou crânio-spinal chez l'homme comprenant l'administration d'une quantité efficace d'une héparine de bas poids moléculaire.

## Revendications

1. Utilisation d'une héparine de bas poids moléculaire pour la préparation d'un médicament réduisant d'au moins 40 pourcent la taille du trauma cérébral et utile pour la prévention et le traitement du trauma du système nerveux central.

2. Utilisation selon la revendication 1 pour la prévention et le traitement du trauma spinal.

3. Utilisation selon la revendication 1 pour la prévention et le traitement du trauma crânien

4. Utilisation selon la revendication 1 pour la prévention et le traitement du trauma crânio-spinal

5. Utilisation selon la revendication 1 pour laquelle l'héparine de bas poids moléculaire a un poids moléculaire moyen compris entre 1000 et 10000 daltons.

6. Utilisation selon la revendication 1 pour laquelle l'héparine de bas poids moléculaire a un poids moléculaire moyen compris entre 1500 et 6000 daltons.

7. Utilisation selon la revendication 1 pour laquelle l'héparine de bas poids moléculaire a un poids moléculaire moyen compris entre 4000 et 5000 daltons.

8. Utilisation selon l'une des revendications 1 à 4 pour laquelle l'héparine de bas poids moléculaire est constituée d'oligosaccharides ayant un acide 2-O-sulfo-4-énopyranosuronique à l'une de leurs extrêmités.

9. Utilisation selon l'une des revendications 1 à 5 pour laquelle l'héparine de bas poids moléculaire est obtenue par dépolymérisation d'un ester de l'héparine au moyen d'une base.

10. Utilisation selon l'une des revendications 1 à 7 pour laquelle l'héparine de bas poids moléculaire est l'énoxaparine (DCI).

11. Utilisation selon l'une des revendications 1 à 7 pour laquelle l'héparine de bas poids moléculaire la nadroparine (DCI).

12. Utilisation selon l'une des revendications 1 à 7 pour laquelle l'héparine de bas poids moléculaire est la parnaparine (DCI).

13. Utilisation selon l'une des revendications 1 à 7 pour laquelle l'héparine de bas poids moléculaire est la reviparine (DCI).

14. Utilisation selon l'une des revendications 1 à 7 pour laquelle l'héparine de bas poids moléculaire est la daltéparine (DCI).

15. Utilisation selon l'une des revendications 1 à 7 pour laquelle l'héparine de bas poids moléculaire est la tinzaparine (DCI).

16. Utilisation selon l'une des revendications 1 à 7 pour laquelle l'héparine de bas poids moléculaire est la danaparoide (DCI).

17. Utilisation selon l'une des revendications 1 à 7 pour laquelle l'héparine de bas poids moléculaire est l'ardeparine (DCI).

18. Utilisation selon l'une des revendications 1 à 7 pour laquelle l'héparine de bas poids moléculaire est la certoparine (DCI).

19. Utilisation selon l'une des revendications 1 à 7 pour laquelle l'héparine de bas poids moléculaire est le CY222.

20. Utilisation selon l'une des revendications 1 à 7 pour laquelle l'héparine de bas poids moléculaire est le SR90107/ORG31540.
